# EUROPEAN PATENT SPECIFICATION

(11) **EP 3 535 786 B1**
(45) Date of publication and mention of the grant of the patent: **13.04.2022**
(21) Application number: 17861177.8
(22) Date of filing: 07.09.2017
(51) Int. Cl.: H01L 39/14

(54) **SUPERCONDUCTING COIL CONFIGURATION**
SUPRALEITENDE SPULENKONFIGURATION
CONCEPTION DE BOBINE SUPRACONDUCTRICE

(30) Priority: 03.11.2016 US 201615342632
(43) Date of publication of application: 11.09.2019
(73) Proprietor: Mevion Medical Systems, Inc., Littleton, MA 01460 (US)
(72) Inventor: SOBCZYNSKI, Stanislaw P., Boxford Massachusetts 01921 (US); AIZED, Dawood, Grafton Massachusetts 01519 (US)
(74) Representative: Peterreins Schley
(86) International application number: PCT/US2017/050565
(87) International publication number: WO 2018/125315

(56) References cited:
- EP-A1- 2 169 735
- EP-A1- 2 642 543
- EP-A2- 1 467 418
- EP-A2- 2 448 029
- US-A- 4 743 713
- US-A1- 2010 031 494
- US-A1- 2014 094 639

## Description

### TECHNICAL FIELD

This disclosure relates generally to a superconducting coil configuration.

### BACKGROUND

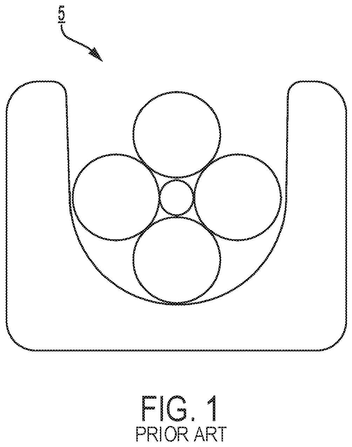

A superconducting magnet generates a magnetic field by passing current through one or more superconducting coils. A known superconducting coil configuration includes, in each integrated conductor, four superconducting strands made from niobium-tin (Nb3 Sn) wound around a non-superconducting copper (Cu) center strand, in the known superconducting coil configuration, each superconducting strand has a diameter of about 0.8 millimeters (mm), and the center strand has a diameter of about 0.33mm. An example of the known four-superconducting-strand implementation 5 is shown in Fig. 1.

Defects in one or more of the superconducting strands can adversely affect critical current (maximum superconductor current at given temperature and (B) field). In some cases, this can affect the operation of the magnet itself. Such defects may include, but are not limited to, breakage or cracks in the strands. The strands may be particularly susceptible to damage at thin points in a strand.

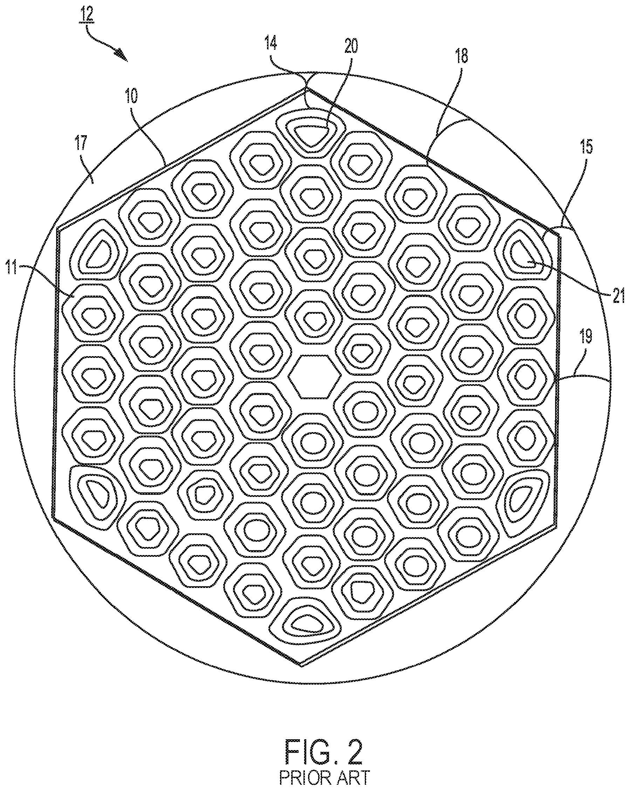

More specifically, each strand of the coil may be extruded from a billet. The billet includes a copper structure comprised of holes filled with a superconducting filaments, such as Nb3 Sn. In the known configuration, there are 61 filaments in the strand. To produce a superconducting strand, the billet is stretched longitudinally using various processes until its diameter is in the sub-millimeter range. The filaments are arranged in a hexagonal pattern. Referring to Fig. 2, the hexagonal pattern 10 of filaments 1 1 is preserved during extrusion, resulting in a superconducting strand 12 having a cross-section like that shown.

As also shown in Fig. 2, certain parts 14, 15 of the resulting strand have thinner copper structure 17 than other parts 18, 19 of the strand. This typically occurs at inflection points, such as inflection points 20, 21, in the hexagonal pattern. Those thinner parts 14, 15 of the strand may be more susceptible to breakage than the thicker parts 18, 19 of the strand. EP 1 467 418 A2, EP 2 139 735 A1, EP 2 642 543 A1, US 4 743 713 A, US 2010/031494 A1, and EP 2 448 029 A2 disclose superconducting coils.

### SUMMARY

The present invention relates to a superconducting coil as set out in claim 1 and a particle therapy system as set out in claim 15. Other embodiments are described in the dependent claims.

The at least six strands may be six strands. The core may comprise a strand having a different diameter than the at least six strands. The core may comprise a strand having a same diameter as the at least six strands. The core may comprise a strand having a diameter that is less than 0.8mm; the core may comprise a strand having a diameter that is less than 0.7mm; or the core may comprise a strand having a diameter that is less than 0.6mm. Each strand may have a cross-section comprised of filaments comprising the superconducting material. The filaments may be within a geometric pattern encompassing multiple inflection points. One or more of the filaments may be absent at each of the multiple inflection points

An example superconducting coil is comprised of superconducting strands. A superconducting strand has a structure comprising a conductive support having filaments that extend longitudinally through the conductive support. The filaments comprise superconducting material, in a cross-section of the superconducting strand, the filaments are within a geometric pattern comprised of multiple inflection points. One or more of the filaments is absent at each of the multiple inflection points. The example superconducting coil may include one or more of the following features, either alone or in combination.

The geometric pattern may comprise a hexagonal shape. A filament may be absent at each inflection point of the hexagonal shape. A number of the one or more filaments may be 54. The conductive support may comprise copper. The strand may be part of an integrated conductor. The integrated conductor may comprise: a core; and the superconducting strand wound around the core along with other superconducting strands such that a total number of superconducting strands wound around the core is at least six. The core may comprise a center strand having a different diameter than the at least six strands. The core may comprise a center strand having a same diameter as the at least six strands. The same diameter may be a diameter that is less than 0.7mm.

Two or more of the features described in this disclosure, including those described in this summary section, may be combined to form implementations not specifically described herein.

Control of the various systems described herein, or portions thereof, may be implemented via a computer program product that includes instructions that are stored on one or more non-transitory machine-readable storage media, and that are executable on one or more processing devices (e.g., microprocessor(s), application-specific integrated circuit(s), programmed logic such as field programmable gate array(s), or the like). The systems described herein, or portions thereof, may be implemented as an apparatus, method, or electronic system that may include one or more processing devices and computer memory to store executable instructions to implement control of the stated functions.

The details of one or more implementations are set forth in the accompanying drawings and the description below. Other features, objects, and advantages will be apparent from the description and drawings, and from the claims.

### DESCRIPTION OF THE DRAWINGS

Fig. 1 is cross-sectional view of a known integrated conductor for use in a superconducting coil.
Fig. 2 is cross-sectional view of a known superconducting strand used in an integrated conductor, and thus a superconducting coil.
Fig. 3 is a cross-sectional view of an example superconducting coil mounted in a reverse bobbin.
Fig. 4 is cross-sectional view of an example integrated conductor having a six-by-one (6×1) configuration for use in a superconducting coil.
Fig. 5 is cross-sectional view of an example superconducting strand used in an integrated conductor, and thus a superconducting coil.
Fig. 6 is a cut-away view of part of an example particle accelerator that uses a superconducting coil of the type described herein.
Figs. 7 and 8 are perspective views of part of a particle therapy system that includes a particle accelerator that uses a superconducting coil of the type described herein.

Like reference symbols in the various drawings indicate like elements.

### DETAILED DESCRIPTION

In general, a superconductor is an element or metallic alloy which, when cooled below a threshold temperature, loses most, if not all, electrical resistance. As a result, current flows through the superconductor substantially unimpeded. Superconducting coils, therefore, are capable of conducting much larger current than ordinary wires of the same size. Because of the high amounts of current that they are capable of conducting, superconducting coils are particularly useful in electromagnetic applications. However, the superconducting coils described herein are not limited to use in electromagnetic applications, and may be used in any appropriate context.

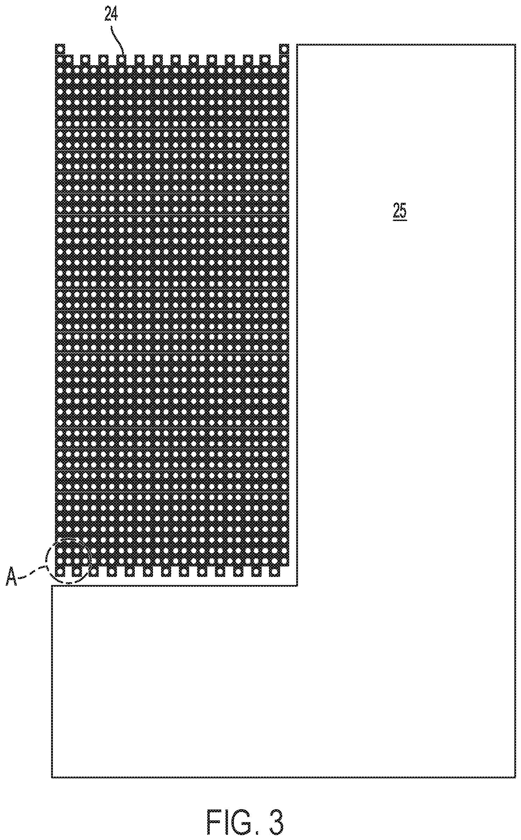

An example superconducting coil is formed by winding integrated conductors to produce a coil having an appropriate size. A cross-section of an example superconducting coil 24 comprised of multiple wound integrated conductors and positioned against a reverse bobbin 25 is shown in Fig. 3. In some implementations, each integrated conductor includes a conductive core and at least six strands wound (e.g., cabled), or twisted, around the core. The strands wound around the core are referred to herein as outer strands, since they are outside the core, in some implementations, both the core and the outer strands include superconducting material and, therefore, can achieve superconductivity when the coil is below the threshold temperature of the superconducting material. In some implementations, the outer strands include superconducting material and the core is made of a non-superconducting material (which may be electrically and thermally conductive). As a result, when the coil is below the threshold temperature, only the outer strands can achieve superconductivity, but the core cannot.

The superconductivity of the strands is also affected by the current passing through the strands. That is, if the current exceeds a specified amount, known as the critical current, the strands will no longer be superconducting.

The superconducting material includes niobium-tin/triniobium-tin (Nb3 Sn), either alone or in combination with, niobium-titanium (NbTi), vanadium- gallium (V3 Ga), bismuth strontium calcium copper oxide (BSCCO), yttrium barium copper oxide (YBCO), or magnesium diboride (MgB2). A non-superconducting material may is included in the core comprising copper. For example, in some implementations, the core may be a copper (CU) conductor.

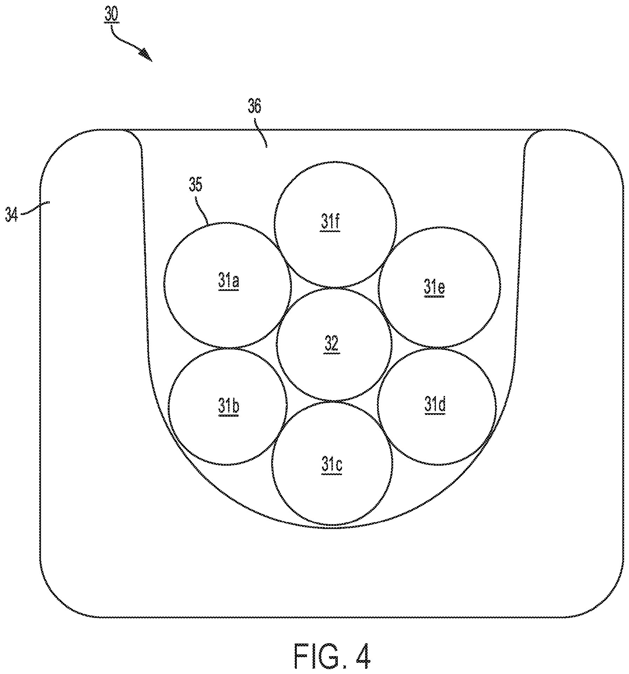

Fig. 4 shows a cross-section of an example integrated conductor 30. As shown, in this example, integrated conductor 30 contains six outer strands 31a to 31f cabled (e.g., wound) around a center strand 32 (the core). This configuration is referred to herein as a 6×1 structure. A characteristic of the 6x1 structure is that each strand has the same diameter. That is, the center strand 32 has the same diameter as each of the outer strands 31a to 31 f, and each of the outer strands 31 a to 31 f has the same diameter. This configuration can contribute to the stability of the cabled superconductors, used to fabricate the integrated conductor, as described herein. In an example, every strand, including the center strand, has a diameter of 0.64mm; however, that value may be different in other implementations, as indicated herein.

The integrated conductor also includes a channel 34. Channel 34 may be made of copper or any other appropriate, e.g., electrically conductive, material. The cabled strands - in this example, 6×1 structure 35 - fit tightly within channel 34, as shown. The channel is then filled with solder 36 or other appropriate conductive material to produce the integrated conductor. The composition of the filled substance (e.g., solder) may stabilize the coil, both thermally and structurally.

As noted above, in some implementations, the core is a non-superconducting strand, which may have the same, or about the same, geometry as the outer strands, but a different composition. For example, the non-superconducting strand may be made of copper or a copper alloy, and include no superconducting material. Accordingly, in such implementations, the superconducting coil includes six strands that are capable of superconductivity, and one strand that is not capable of superconductivity. Although, for the same-size strands, the current-carrying capacity of a six-superconducting-strand implementation is less than that of a seven-superconducting-strand implementation (for two integrated conductors having the same cross-sectional area), the six-superconducting-strand implementation may have more structural and thermal stability than the seven-superconducting-strand implementation. That is, since the copper itself is not a superconductor, the copper provides structural stability, and a low resistance path for large currents in case of a quench, e.g., when the temperature of the coil rises above the superconductivity threshold resulting in loss of superconductivity or the current through the superconducting coil rises above the coil's critical current resulting in loss of superconductivity. Furthermore, during operation, the copper core - which may be both electrically and thermally conductive - can act as a thermal conduit to promote cooling and to improve heat transfer during quench. Thus, increasing the amount of copper in the channel cross-section relative to solder promotes thermal stability in the integrated conductor.

Although a 6x1 implementation is described with respect to the figures, any appropriate number of strands may be included in integrated conductors of the superconducting coil described herein. For example, implementations of the superconducting coil may include five, six, seven, eight, nine, ten, eleven, twelve, etc. strands wound around a core in an integrated conductor. In implementations where there are more or less than six outer strands, the core will have a diameter that is different than (e.g., larger or smaller than) the diameter of the outer strands. The core itself may be a single strand or include multiple strands that are wound, or twisted, together. As noted the core may include superconducting material or be comprised solely of non-superconducting material.

In any case, an increase in the number of superconducting strands over the known four-superconducting-strand implementation may improve reliability relative to the known four-superconducting-strand implementation . For example, the increase in the number of strands causes current flow to be distributed across more strands than in the known four-superconducting-strand implementation. As a result, a defect in an individual strand will have less of an overall impact on the current flow than in the known four-superconducting-strand implementation. That is, because each strand carries less of the total current, the failure of one strand may be more easily compensated for by the other strands.

In some implementations, the increase in the number of strands does not substantially increase the size of the integrated conductor having the configurations described herein. For example, the integrated conductor may include strands having diameters of 0.7mm or less, 0.6mm or less, 0.5mm, 0.4mm or less, 0.3mm or less, and so forth. Accordingly, although there may be more strands than in the known configuration (having four strands each of 0.8mm diameter), the strands are smaller and, therefore, the cross-sectional area of the integrated conductor may remain about the same as in the known 0.8mm strand configuration. In the particular example of Fig. 4, the overall cross-sectional area of integrated conductor 30 is identical to, or about the same as, the overall cross-sectional area of the known implementation of Fig. 1. Because the cross-sectional area of the integrated conductor has not changed in some implementations, the size (e.g., cross-sectional area) of the coil formed by winding multiple such integrated conductors may also not change.

The following table compares example coil parameters for the known four-superconducting-strand implementation (4-strand design) to an example of the six-superconducting-strand implementation (6-strand design) and an example of the seven-superconducting-strand implementation (7-strand design). The parameters for the six-superconducting-strand implementation and the seven-superconducting-strand implementation are examples only, and this disclosure encompasses other designs having different values of these parameters.

| PARAMETER | 4-STRAND DESIGN | 6-STRAND DESIGN | 7-STRAND DESIGN |
|---|---|---|---|
| STRAND DIAMETER (CROSS-SECTION IN MM) | 0.804 | 0.640 | 0.640 |
| FRACTION OF COPPER (CU) IN STRAND (IN PERCENTAGE) | 40 | 40 | 40 |
| FRACTION OF COPPER (CUI) IN CORE (IN PERCENTAGE) | 100 | 100 | 40 |
| CORE DIAMETER (CROSS-SECTION IN mm) | 0.320 | 0.640 | 0.640 |
| COPPER AREA TOTAL (CROSS-SECTION IN mm²) | 5.284 | 5.485 | 5.292 |
| SUPERCONDUCTOR AREA TOTAL (CROSS-SECTION IN mm²) | 1.218 | 1.158 | 1.351 |
| CONDUCTOR AREA TOTAL (CROSS-SECTION IN mm²) | 6.502 | 6.643 | 6.643 |
| AREA FILLED WITH SOLDER (CROSS-SECTION IN mm²) | 1.562 | 1.422 | 1.422 |
| COPPER/SUPERCONDUCTOR RATIO | 4.336 | 4.736 | 3.917 |
| I_OPP / I_C (OPERATIONAL/CRITICAL CURRENT (PERFORMANCE MARGIN) | 0.569 | 0.598 | 0.513 |
| HOT SPOT TEMPERATURE (ADIABATIC, COPPER RRR = 100) (IN °KELVIN) | 262 | 252 | 263 |

As shown in the table above, the known four-superconducting-strand implementation actually has a larger cross-sectional superconducting area than the example six-superconducting-strand implementation. Consequently, the known four-superconducting-strand implementation may have greater current-carrying capacity than the example six-superconducting-strand implementation (for coils having about the same cross-sectional area). However, the example six-superconducting-strand implementation has advantages, as described herein, in terms of stability and operational reliability. The example seven-superconducting-strand implementation has a larger cross-sectional superconducting area than the known four-superconducting-strand implementation and thus may have greater current-carrying capacity than the known four-superconducting-strand implementation (for conductors having about the same cross-sectional area).

As explained above, the integrated conductors described herein are wound to produce a superconducting coil. The benefits described for the different integrated conductors inure to the superconducting coil produced therefrom. That is, because a superconducting coil is formed by winding multiple integrated conductors, the enhanced stability, reliability, current capacity, or other benefits of the integrated conductors also apply to the resulting superconducting coil.

Superconducting coil implementations described herein may be used in a superconducting magnet. For example, for an integrated conductor having a six-strand configuration, each superconducting strand may be configured to support a critical current of 557 Amperes (A) at operating conditions of about 4.2°K and about 11 Tesla (T) magnetic field. For a six-strand implementation having current carrying capacity of 2000A, each strand carries about 333A. For a six-strand implementation having current carrying capacity of 3000A, each strand carries about 500A. An example seven-superconducting-strand implementation may improve the electromagnetic performance of the magnet for reasons described herein. Referring to the table above, a lower hot spot temperature during a magnet quench implies that the six-superconducting-strand implementation is thermally more stable. On the other hand, a magnet wound with a coil that employs the seven-superconducting-strand implementation may operate at a lower fraction of the critical current.

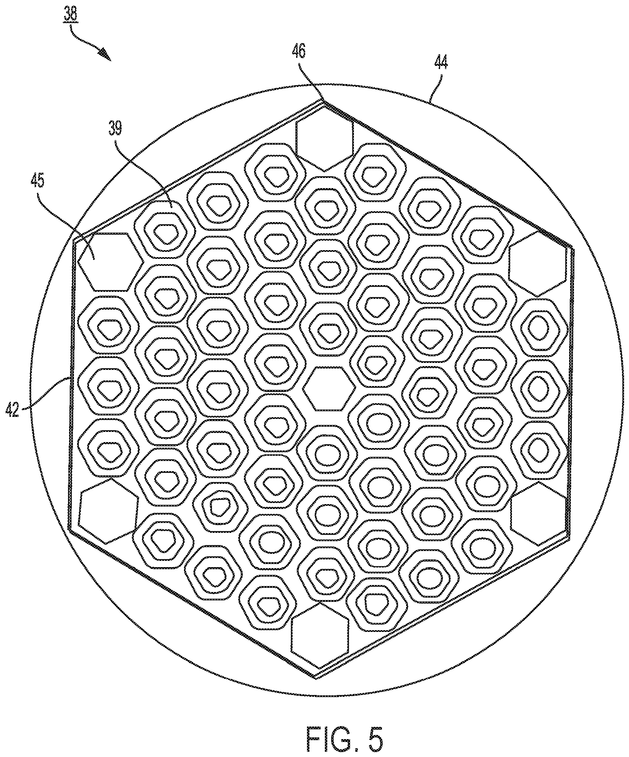

Fig. 5 shows the cross-section of a strand 38, which is a component of the integrated conductors, and thus the superconducting coils, described herein. Strand 38 may be an outer strand or a core strand. As explained herein, strand 38 is extruded from a billet having a cross-sectional configuration that is that same as, or substantially the same as, that of the strand. That is, during extrusion, the cross-sectional configuration of the original billet - in this example, a hexagonal arrangement of superconducting filaments surrounded by copper - is preserved in the resulting extruded strand. The billet used to produce strand 38 include holes that extend longitudinally therethrough. The holes are filled with a superconducting material to produce the filaments 39 of Fig. 5, which extend longitudinally through the billet and, thus, through a strand extruded therefrom. Each filament may be, or include, Nb₃Sn. In some implementations, each filament is comprised of the same type of superconducting material.

As shown in Fig. 5, filaments 39 are arranged so that they are within a geometric pattern, in the example of Fig. 5, the filaments are arranged within a hexagonal pattern, in which the filaments are encompassed by a hexagon 42 (which is within the circumference 44 of the circular cross-section of strand 38). However, in other implementations, the filaments may be arranged within other geometric patterns, e.g., within a square, a pentagon, a hexagon, and so forth. The geometric pattern used may be dependent upon the number of filaments to be included in the strand. In this example, 54 (fifty-four) filaments may be included within the strand within the bounds of the hexagon. As explained below, some strands having a substantially hexagonal pattern of Fig. 5 (or other geometric pattern) may include the fewer filaments, which may result in a strand configuration having an enhanced resistance to cracking or breaking.

More specifically, as shown in Fig. 5, the geometric pattern - in this case, the hexagonal pattern - includes multiple inflection points, such as 45 and 46. Other geometric patterns - both polygonal or non-polygonal, such as ellipses, stadiums, and ovals - may contain similar inflection points. An inflection point includes a point of the geometric pattern at which a change in a direction of curvature occurs. Referring to Fig. 2, as explained above, in the known configuration, at about the locations of inflection points of hexagon 10, the amount of copper in the strand is less than (e.g., the copper is thinner than) at other points along the hexagon, such as locations 18 and 19, which are between inflection points. Because there is less copper at the inflection points, there is less structural support at those points and, therefore, the strand may be more susceptible to breakage or other damage at those points.

The strand of Fig. 5, however, does not include filaments at all or some of the inflection points of hexagon 42. In Fig. 5, filaments are not included at about the locations of inflection points of hexagon 42, such as points 45 and 46. Thus, the thickness of copper at the inflection points of hexagon 42 is increased relative to implementations, such as that shown in Fig. 2, that include filaments at the inflection points. In Fig. 5, filaments are absent at each inflection point of hexagon 42; however, in some implementations where filaments are missing at inflection points, one or more of the other inflection points may contain a filament. In any case, in some implementations, strands without filaments at all or some of its inflection points may be less susceptible to cracking or breaking at those points. That is, the additional copper at those points may increase the structural and thermal stability of the strand at those points, making it less likely that there will be breaks or cracks at those points.

As noted above, in some implementations, filaments are not included at least at inflection points, such as 45 and 46. In addition, one or more additional filaments at the interior or perimeter of the hexagon may be absent as well. In an implementation, such as that shown in Fig. 5, there are 54 filaments in an individual strand, with filaments being absent at the inflection points of the geometric pattern - in this example, the hexagon. The hexagonal pattern thus encompasses (e.g., is around) the 54 filaments. In some implementations, the 54 filaments may be sized and arranged to include enough superconducting material so that the resulting strand carries sufficient current to support the six-superconducting-strand implementation and the seven-superconducting-strand implementation described herein. In some implementations, geometry may be modified to increase the current-carrying capacity of the superconducting coil.

To form a strand, such as strand 38, a billet having holes (in this example, 54 holes) filled with superconducting filament is extruded (e.g., stretched longitudinally) until it has the appropriate, sub-millimeter, diameter. In some implementations, the cross-sectional structure of the strand, however, is preserved during the extrusion process. As a result, the strand retains the cross-sectional structure of Fig. 5, including at diameters of less than 0.8mm, as described herein.

In some implementations, the filaments are arranged in a strand in a geometric pattern so that the amount of conductive support (e.g., copper) between each filament and a circumference 44 of the cross-section varies is increased relative to conventional implementations. This may be achieved, e.g., by excluding filaments at the inflection points of a geometric pattern as described above, or though other arrangement of filaments in the strand. For example, the filaments may be positioned at concentric circles that expand in diameter outwardly from a center. Any appropriate configuration of filaments in the strand may be used to increase the amount of conductive support.

The superconducting coils described herein may be used in any appropriate context. As noted, the example superconducting coil may incorporated into an electromagnet that is a component of a particle accelerator, such as a synchrocyclotron, of a particle therapy system.

A particle accelerator system is a proton or ion therapy system, which contains a magnet that employs a superconducting coil of the type described herein. The particle therapy system includes a particle accelerator - in this embodiment a synchrocyclotron - mounted on a gantry. The gantry enables the accelerator to be rotated around a patient position to allow a particle beam from the particle accelerator to hit any arbitrary treatment area in the patient. Likewise, the particle accelerator may produce a particle beam having sufficient energy to reach any arbitrary target within the patent from an appropriate rotational position. As a result of rotation of the gantry around the patient, the particle accelerator also rotates around the patient position.

In some implementations, the synchrocyclotron has a high magnetic field superconducting electromagnetic structure. Because the bend radius of a charged particle of a given kinetic energy is reduced in direct proportion to an increase in the magnetic field applied to it, the high magnetic field superconducting magnetic structure permits the accelerator to be made relatively small and light. The synchrocyclotron uses a magnetic field that is uniform in rotation angle and falls off in strength with increasing radius. In some implementations, such a field shape can be achieved regardless of the magnitude of the magnetic field.

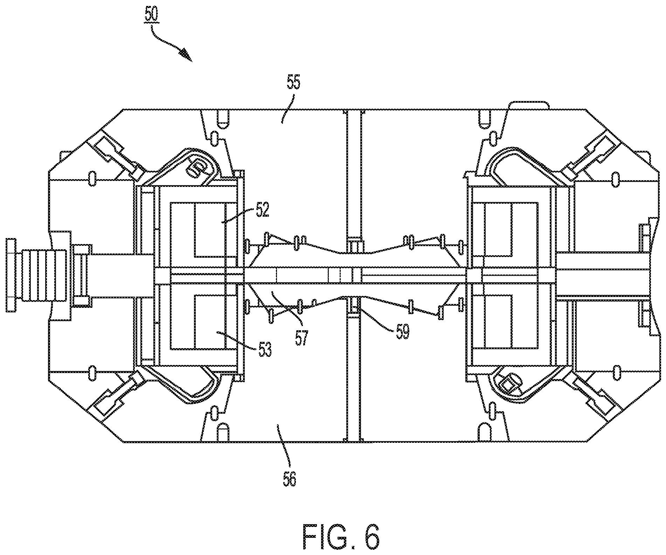

Fig. 6 shows a cross-section of components 50 of a particle accelerator that may employ the superconducting coils described herein. In this embodiment, components 50 of a particle accelerator (e.g., a synchrocyclotron) include, in its magnet, superconducting coils 52 and 53, which are coils having one or more of the features described herein, e.g., with respect to Figs. 3 to 5. Each of the superconducting coils 52, 53 is for conducting a current that generates a magnetic field (B). In an example, a cryostat uses liquid helium (He) to maintain each coil at superconducting temperatures, e.g., around 4 Kelvin (K). Magnetic yokes 55, 56 or smaller magnetic pole pieces (not shown) are located inside the cryostat, and define the shape of a cavity 57 in which particles are accelerated. Magnetic shims (not shown) may pass through the magnetic yokes or pole pieces to change the shape and/or magnitude of the magnetic field in the cavity.

In this implementation, the particle accelerator includes a particle source 59 (e.g., a Penning Ion Gauge - PIG source) to provide an ionized plasma column to the cavity. Hydrogen gas is ionized to produce the plasma column. A voltage source provides a radio frequency (RF) voltage to the cavity to accelerate pulses of particles from the plasma column into the cavity. The magnetic field in the cavity is shaped to cause particles to move orbitally within the cavity. In some implementations, the magnetic field produced by the superconducting coils may be within the range of 4 Tesla (T) to 20T, as explained herein.

As noted, the particle accelerator is a synchrocyclotron. Accordingly, the RF voltage is swept across a range of frequencies to account for relativistic effects on the particles (e.g., increasing particle mass) when accelerating particles from the plasma column. The magnetic field produced by running current through the superconducting coils, together with the shape of the cavity, causes particles accelerated from the plasma column to accelerate orbitally within the cavity. In other implementations, a particle accelerator other than a synchrocyclotron may be used. For example, a cyclotron, a synchrotron, a linear accelerator, and so forth may be substituted for the synchrocyclotron described herein.

In the synchrocyclotron, a magnetic field regenerator ("regenerator") is positioned near the outside of the cavity (e.g., at an interior edge thereof) to adjust the existing magnetic field inside the cavity to thereby change locations (e.g., the pitch and angle) of successive orbits of the particles accelerated from the plasma column so that, eventually, the particles output to an extraction channel that passes through the cryostat. The regenerator may increase the magnetic field at a point in the cavity (e.g., it may produce a magnetic field "bump" of about 2 Tesla or so at an area of the cavity), thereby causing each successive orbit of particles at that point to precess outwardly toward the entry point of an extraction channel until it reaches the extraction channel. The extraction channel receives, from the cavity, particles accelerated from the plasma column and outputs the received particles from the cavity as a particle beam. For example, the extraction channel may contain magnets and other structures to direct the beam out of the particle accelerator and towards a scanning or scattering system.

As noted, the superconducting coils (the main coils) can produce relatively high magnetic fields. The maximum magnetic field generated by a main coil may be within a range of 4T to 20T or more.

Furthermore, the superconducting coils may be used to generate magnetic fields that are within the range of 4T to 20T (or more, or less) that are not specifically listed above. Each superconducting coil may be a superconducting coil of a type described herein with respect to Figs. 3 to 5.

In some implementations, such as the implementations shown in Fig. 6, relatively large ferromagnetic magnetic yokes 55, 56 act as returns for stray magnetic fields produced by the superconducting coils. In some systems, a magnetic shield surrounds the yoke. The return yokes and the shield together act to reduce stray magnetic fields, thereby reducing the possibility that stray magnetic fields will adversely affect the operation of the particle accelerator.

In some implementations, the return yoke and shield may be replaced by, or augmented by, an active return system. An active return system includes one or more active return coils that conduct current in a direction opposite to current through the main superconducting coils. In some implementations, there is an active return coil for each superconducting coil, e.g., two active return coils - one for each main superconducting coil. Each active return coil may also be a superconducting coil that surrounds the outside of a corresponding main superconducting coil concentrically. Each active return coil may thus be a coil of the type described herein, e.g., with respect to Figs. 3 to 5.

As noted, current passes through the active return coils in a direction that is opposite to the direction of current passing through the main coils. The current passing through the active return coils thus generates a magnetic field that is opposite in polarity to the magnetic field generated by the main coils. As a result, the magnetic field generated by an active return coil is able to reduce at least some of the relatively strong stray magnetic field resulting from a corresponding main coil.

The output of the extraction channel of the particle accelerator may include one or more beam shaping elements, such as a scanning system or a scattering system. A scanning system may be used to scan the particle beam across at least part of an irradiation target. A scattering system may be used to scatter the beam prior to application to the irradiation target.

The geometry of the coil is maintained by mounting the coils in the reverse bobbin to exert a restorative force that works against the distorting force produced when the coils are energized. For example, Fig. 3 shows a superconducting coil 24 disposed in a reverse bobbin 25.

The particle accelerator may be a variable-energy particle accelerator. In some implementations, the particle accelerator is configured to vary the energy of the output particle beam by varying the magnetic field in which the particle beam is accelerated. For example, the current may be set to any one of multiple values to produce a corresponding magnetic field. In an implementation, one or more sets of superconducting coils receives variable electrical current to produce a variable magnetic field in the cavity. In some implementations, one set of coils receives a fixed electrical current, while one or more other sets of coils receives a variable current so that the total current received by the coil sets varies. In some implementations, all sets of coils are superconducting. In other implementations, some sets of coils, such as the set for the fixed electrical current, are superconducting, while other sets of coils, such as the one or more sets for the variable current, are non-superconducting. The superconducting coils may be superconducting coils of the type described herein with respect to Figs. 3 to 5.

Generally, the magnitude of the magnetic field is scalable with the magnitude of the electrical current. Adjusting the total electric current of the coils in a predetermined range can generate a magnetic field that varies in a corresponding, predetermined range. In some implementations, a continuous adjustment of the electrical current can lead to a continuous variation of the magnetic field and a continuous variation of the output beam energy. Alternatively, when the electrical current applied to the coils is adjusted in a non-continuous, step-wise manner, the magnetic field and the output beam energy also varies accordingly in a non-continuous (step-wise) manner. The scaling of the magnetic field to the current can allow the variation of the beam energy to be carried out relatively precisely, although sometimes minor adjustment other than the input current may be performed.

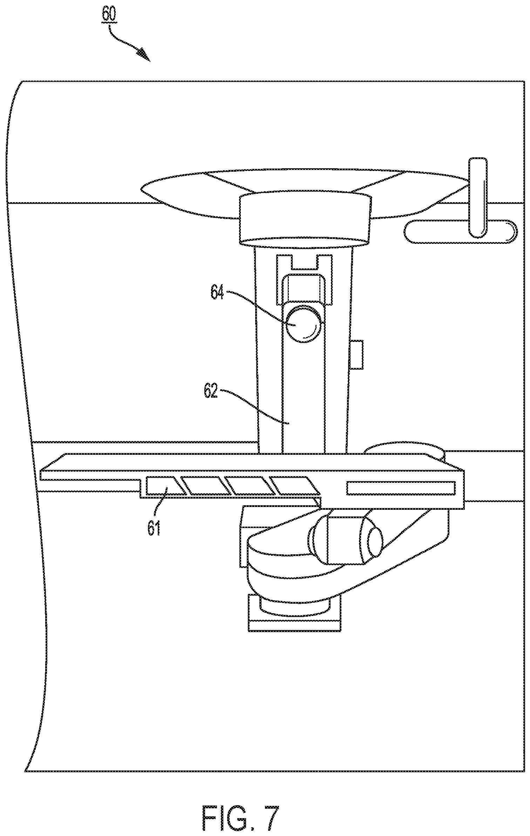

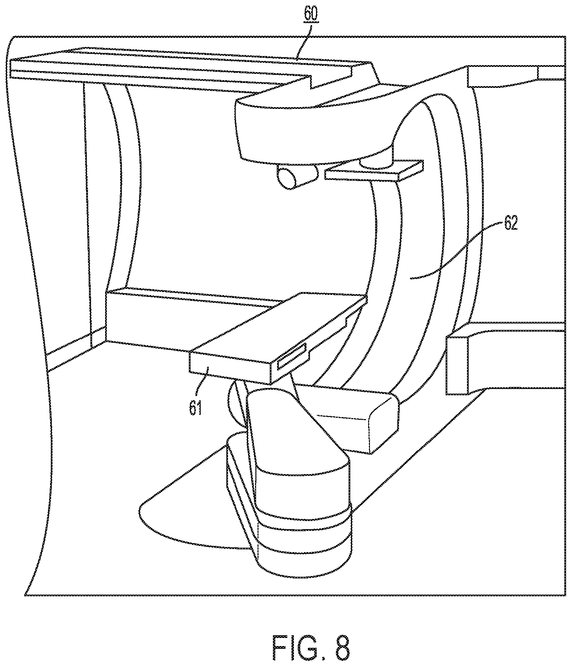

Figs. 7 and 8 show parts of a particle therapy system 60 containing a particle accelerator mounted on a gantry - the synchrocyclotron contain one or more superconducting coils of the type described herein. In some implementations, the gantry (not shown) is steel and has two legs mounted for rotation on two respective bearings that lie on opposite sides of a patient. The gantry may include a steel truss, connected to each of the legs, that is long enough to span a treatment area in which the patient lies and that is attached at both ends to the rotating legs of the gantry. The particle accelerator may be supported by the steel truss. An example of a gantry configuration that may be used is described in U.S. Patent No. 7,728,311 entitled "Charged Particle Radiation Therapy".

As shown in Figs. 7 and 8, the patient position includes a treatment couch 61. An inner gantry 62 moves relative to the treatment couch to direct output 64 of the beam, and to shape the beam, prior to reaching the patient. As shown, the inner gantry is C-shaped, and its movement coincides with movement of the "outer" gantry, on which the synchrocyclotron is mounted.

The control of the outer and inner gantries, the treatment couch, the beam shaping elements, and the synchrocyclotron to perform a therapy session is achieved by appropriate therapy control electronics (not shown). In this regard, control of the particle therapy system described herein and its various features may be implemented using hardware or a combination of hardware and software. For example, a system like the ones described herein may include various controllers and/or processing devices located at various points. A central computer may coordinate operation among the various controllers or processing devices. The central computer, controllers, and processing devices may execute various software routines to effect control and coordination of testing and calibration.

System operation can be controlled, at least in part, using one or more computer program products, e.g., one or more computer program tangibly embodied in one or more non-transitory machine-readable media, for execution by, or to control the operation of, one or more data processing apparatus, e.g., a programmable processor, a computer, multiple computers, and/or programmable logic components.

A computer program can be written in any form of programming language, including compiled or interpreted languages, and it can be deployed in any form, including as a stand-alone program or as a module, component, subroutine, or other unit suitable for use in a computing environment. A computer program can be deployed to be executed on one computer or on multiple computers at one site or distributed across multiple sites and interconnected by a network.

Actions associated with implementing all or part of the operations of the particle therapy system described herein can be performed by one or more programmable processors executing one or more computer programs to perform the functions described herein. All or part of the operations can be implemented using special purpose logic circuitry, e.g., an FPGA (field programmable gate array) and/or an ASIC (application-specific integrated circuit).

Processors suitable for the execution of a computer program include, by way of example, both general and special purpose microprocessors, and any one or more processors of any kind of digital computer. Generally, a processor will receive instructions and data from a read-only storage area or a random access storage area or both. Elements of a computer (including a server) include one or more processors for executing instructions and one or more storage area devices for storing instructions and data. Generally, a computer will also include, or be operatively coupled to receive data from, or transfer data to, or both, one or more machine-readable storage media, such as mass PCBs for storing data, e.g., magnetic, magneto-optical disks, or optical disks. Non-transitory machine-readable storage media suitable for embodying computer program instructions and data include all forms of non-volatile storage area, including by way of example, semiconductor storage area devices, e.g., EPROM, EEPROM, and flash storage area devices; magnetic disks, e.g., internal hard disks or removable disks; magneto-optical disks; and CD-ROM and DVD-ROM disks.

Any "electrical connection" as used herein may imply a direct physical connection or a wired or wireless connection that includes intervening components but that nevertheless allows electrical signals to flow between connected components. Any "connection" involving electrical circuitry that allows signals to flow, unless stated otherwise, is an electrical connection and not necessarily a direct physical connection regardless of whether the word "electrical" is used to modify "connection".

Any two more of the foregoing implementations may be used in an appropriate combination in an appropriate particle accelerator (e.g., a synchrocyclotron). Likewise, individual features of any two more of the foregoing implementations may be used in an appropriate combination.

The coils described herein are not limited to use with a particle therapy system or to use with the example particle therapy systems described herein.

Elements of different implementations described herein may be combined to form other implementations not specifically set forth above. Elements may be left out of the processes, systems, apparatus, etc., described herein without adversely affecting their operation. Various separate elements may be combined into one or more individual elements to perform the functions described herein.

Another implementation of a particle therapy system in which the coils described herein may be implemented is described in U.S. Patent No. 7,728,311 entitled "Charged Particle Radiation Therapy".

Other implementations not specifically described herein are also within the scope of the following claims.

## Claims

1. A superconducting coil (24) comprising:
integrated conductors (30), an integrated conductor comprising:
a core (32) comprising a non-superconducting center strand comprising copper; and
at least six strands (31a - 31f; 38) cabled around the core thereby forming a structure (35), each of the at least six strands comprising superconducting Nb₃Sn;
wherein each of the at least six strands has a cross-section comprised of filaments (39) comprising the superconducting Nb₃Sn, the filaments being within a geometric pattern encompassing multiple inflection points (45, 46), wherein one or more of the filaments is absent at all or some of the multiple inflection points;
wherein the integrated conductor further comprises a channel (34) comprising electrically conductive material; and
wherein the structure is fitted within the channel, and wherein the channel is filled with a conductive material.

2. The superconducting coil of claim 1, wherein the at least six strands is six strands.

3. The superconducting coil of claim 1, wherein the conductive material filled in the channel is solder (36).

4. The superconducting coil of claim 1, wherein the center strand solely comprises non-superconducting material, in particular, copper.

5. The superconducting coil of claim 1, wherein the core comprises a strand having a different diameter than the at least six strands.

6. The superconducting coil of claim 1, wherein the core comprises a strand having a same diameter as the at least six strands.

7. The superconducting coil of claim 1, wherein the core comprises a strand having a diameter that is less than 0.8mm.

8. The superconducting coil of claim 1, wherein the core comprises a strand having a diameter that is less than 0.7mm.

9. The superconducting coil of claim 1, wherein the core comprises a strand having a diameter that is less than 0.6mm.

10. The superconducting coil of claim 1, wherein one or more of the filaments is absent at each of the multiple inflection points.

11. The superconducting coil of any one of the previous claims, wherein the geometric pattern is arranged within a circumference (44) of the cross-section of the respective strand, wherein each strand comprises a conductive support having the filaments which extend longitudinally through the conductive support.

12. The superconducting coil of any one of the previous claims, wherein the filaments are arranged so that the amount of conductive support between each filament and a circumference (44) of the cross-section is increased.

13. The superconducting coil of any one of the previous claims, wherein the geometric pattern comprises a hexagonal shape, and optionally, wherein a filament is absent at each inflection point of the hexagonal shape.

14. The superconducting coil of any one of claims 11 or 12, wherein the conductive support comprises copper.

15. A particle therapy system (60) comprising:
a synchrocyclotron; and
a gantry (62) on which the synchrocyclotron is mounted to rotate at least part-way around a patient position to position the synchrocyclotron relative to a treatment area of the patient;
wherein the synchrocyclotron comprises:
a magnet comprising coils to receive electrical current and to generate a magnetic field in response to the electrical current, the magnetic field for causing the particles to move orbitally within a cavity (57) at an energy that corresponds to the electrical current, the coils being superconducting coils according to any one of the previous claims; and
an extraction channel to receive the particles from the cavity and to output the particles received from the cavity.

## Patentansprüche

1. Supraleitende Spule (24), umfassend:
integrierte Leiter (30), wobei ein integrierter Leiter umfasst:
einen Kern (32), der einen nicht-supraleitenden Mittelstrang umfasst, der Kupfer umfasst; und
mindestens sechs Stränge (31a - 31f; 38), die um den Kern herum verkabelt sind, wodurch eine Struktur (35) gebildet wird, wobei jeder der mindestens sechs Stränge supraleitendes Nb₃Sn umfasst;
wobei jeder der mindestens sechs Stränge einen Querschnitt aufweist, der aus Filamenten (39) zusammengesetzt ist, welche das supraleitende Nb₃Sn umfassen, wobei die Filamente innerhalb eines geometrischen Musters vorliegen, das mehrere Wendepunkte (45, 46) umschließt, wobei ein oder mehrere der Filamente an allen oder einigen der mehreren Wendepunkte fehlt bzw. fehlen;
wobei der integrierte Leiter des Weiteren einen Kanal (34) umfasst, der elektrisch leitendes Material umfasst; und
wobei die Struktur in den Kanal eingepasst ist, und wobei der Kanal mit einem leitfähigen Material gefüllt ist.

2. Supraleitende Spule nach Anspruch 1, wobei die mindestens sechs Stränge sechs Stränge sind.

3. Supraleitende Spule nach Anspruch 1, wobei das in den Kanal gefüllte leitfähige Material Lot (36) ist.

4. Supraleitende Spule nach Anspruch 1, wobei der Mittelstrang nur nicht-supraleitendes Material umfasst, insbesondere Kupfer.

5. Supraleitende Spule nach Anspruch 1, wobei der Kern einen Strang mit einem anderen Durchmesser als die mindestens sechs Stränge umfasst.

6. Supraleitende Spule nach Anspruch 1, wobei der Kern einen Strang mit demselben Durchmesser wie die mindestens sechs Stränge umfasst.

7. Supraleitende Spule nach Anspruch 1, wobei der Kern einen Strang mit einem Durchmesser kleiner als 0,8 mm umfasst.

8. Supraleitende Spule nach Anspruch 1, wobei der Kern einen Strang mit einem Durchmesser kleiner als 0,7 mm umfasst.

9. Supraleitende Spule nach Anspruch 1, wobei der Kern einen Strang mit einem Durchmesser kleiner als 0,6 mm umfasst.

10. Supraleitende Spule nach Anspruch 1, wobei ein oder mehrere der Filamente an jedem der mehreren Wendepunkte fehlt bzw. fehlen.

11. Supraleitende Spule nach einem der vorhergehenden Ansprüche, wobei das geometrische Muster innerhalb eines Umfangs (44) des Querschnitts des jeweiligen Strangs angeordnet ist, wobei jeder Strang einen leitfähigen Träger umfasst, der die Filamente umfasst, die sich in Längsrichtung durch den leitfähigen Träger hindurch erstrecken.

12. Supraleitende Spule nach einem der vorhergehenden Ansprüche, wobei die Filamente so angeordnet sind, dass die Menge an leitfähigem Träger zwischen jedem Filament und einem Umfang (44) des Querschnitts erhöht ist.

13. Supraleitende Spule nach einem der vorhergehenden Ansprüche, wobei das geometrische Muster eine hexagonale Form umfasst, und wobei gegebenenfalls ein Filament an jedem Wendepunkt der hexagonalen Form fehlt.

14. Supraleitende Spule nach einem der Ansprüche 11 oder 12, wobei der leitfähige Träger Kupfer umfasst.

15. Partikeltherapiesystem (60), umfassend:
ein Synchrozyklotron; und
eine Gantry (62), auf welcher das Synchrozyklotron montiert ist, um mindestens teilweise um eine Patientenposition herum zu rotieren, um das Synchrozyklotron relativ zu einem Behandlungsbereich des Patienten zu positionieren;
wobei das Synchrozyklotron umfasst:
einen Magneten, der Spulen umfasst, um elektrischen Strom anzunehmen und ein Magnetfeld in Reaktion auf den elektrischen Strom zu generieren, wobei das Magnetfeld bewirken soll, dass sich die Partikel im Orbit innerhalb eines Hohlraums (57) mit einer Energie bewegen, welche dem elektrischen Strom entspricht, wobei die Spulen supraleitende Spulen gemäß einem der vorhergehenden Ansprüche sind; und
einen Extraktionskanal zum Annehmen der Partikel von dem Hohlraum und zum Ausgeben der von dem Hohlraum angenommenen Partikel.

## Revendications

1. Bobine supraconductrice (24) comprenant :
des conducteurs intégrés (30), un conducteur intégré comprenant :
un noyau (32) comprenant un brin central non supraconducteur comprenant du cuivre ; et
au moins six brins (31a - 31f; 38) câblés autour du noyau formant ainsi une structure (35), chacun des au moins six brins comprenant du Nb₃Sn supraconducteur ;
chacun des au moins six brins ayant une section transversale constituée de filaments (39) comprenant le Nb₃Sn supraconducteur, les filaments étant à l'intérieur d'un motif géométrique englobant de multiples points d'inflexion (45, 46), un ou plusieurs des filaments étant absents à tous ou à certains des multiples points d'inflexion ;
le conducteur intégré comprenant en outre un canal (34) comprenant un matériau électriquement conducteur ; et
la structure étant ajustée à l'intérieur du canal, et le canal étant rempli d'un matériau conducteur.

2. Bobine supraconductrice selon la revendication 1, les au moins six brins étant six brins.

3. Bobine supraconductrice selon la revendication 1, le matériau conducteur rempli dans le canal étant un matériau de soudure (36).

4. Bobine supraconductrice selon la revendication 1, le brin central comprenant uniquement un matériau non supraconducteur, en particulier du cuivre.

5. Bobine supraconductrice selon la revendication 1, le noyau comprenant un brin ayant un diamètre différent de celui des au moins six brins.

6. Bobine supraconductrice selon la revendication 1, le noyau comprenant un brin ayant un même diamètre que les au moins six brins.

7. Bobine supraconductrice selon la revendication 1, le noyau comprenant un brin ayant un diamètre inférieur à 0,8 mm.

8. Bobine supraconductrice selon la revendication 1, le noyau comprenant un brin ayant un diamètre inférieur à 0,7 mm.

9. Bobine supraconductrice selon la revendication 1, le noyau comprenant un brin ayant un diamètre inférieur à 0,6 mm.

10. Bobine supraconductrice selon la revendication 1, un ou plusieurs des filaments étant absents à chacun des multiples points d'inflexion.

11. Bobine supraconductrice selon l'une quelconque des revendications précédentes, le motif géométrique étant agencé à l'intérieur d'une circonférence (44) de la section transversale du brin respectif, chaque brin comprenant un support conducteur ayant les filaments qui s'étendent longitudinalement à travers le support conducteur.

12. Bobine supraconductrice selon l'une quelconque des revendications précédentes, les filaments étant agencés de telle sorte que la quantité de support conducteur entre chaque filament et une circonférence (44) de la section transversale est augmentée.

13. Bobine supraconductrice selon l'une quelconque des revendications précédentes, le motif géométrique comprenant une forme hexagonale, et éventuellement, un filament étant absent à chaque point d'inflexion de la forme hexagonale.

14. Bobine supraconductrice selon l'une quelconque des revendications 11 ou 12, le support conducteur comprenant du cuivre.

15. Système de thérapie par particules (60) comprenant :
un synchrocyclotron ; et
un portique (62) sur lequel le synchrocyclotron est monté pour tourner au moins en partie autour d'une position du patient pour positionner le synchrocyclotron par rapport à une zone de traitement du patient ;
le synchrocyclotron comprenant :
un aimant comprenant des bobines pour recevoir un courant électrique et pour générer un champ magnétique en réponse au courant électrique, le champ magnétique étant destiné à provoquer le déplacement orbital des particules à l'intérieur d'une cavité (57) à une énergie qui correspond au courant électrique, les bobines étant des bobines supraconductrices selon l'une quelconque des revendications précédentes ; et
un canal d'extraction pour recevoir les particules provenant de la cavité et pour émettre en sortie les particules reçues de la cavité.
